(19) Europäisches Patentamt
European Patent Office
Office européen des brevets

(11) EP 0 682 015 B1

(12) **EUROPEAN PATENT SPECIFICATION**

(45) Date of publication and mention of the grant of the patent:
**22.08.2001 Bulletin 2001/34**

(51) Int Cl.7: **C07D 211/70**, C07D 401/12, C07D 409/04, C07D 409/14, C07D 413/06, C07D 413/14, C07D 417/06, A61K 31/445

(21) Application number: **95302647.3**

(22) Date of filing: **20.04.1995**

(54) **Piperidine derivates and anti-platelet agents containing the same**

Piperidin-Derivate und diese enthaltende Antiplättchen-Agents

Dérivés de la pipéridine et agents anti-plaquettaires les contenant

(84) Designated Contracting States:
**AT BE CH DE DK ES FR GB GR IE IT LI LU MC NL PT SE**

(30) Priority: **20.04.1994 JP 8149994**

(43) Date of publication of application:
**15.11.1995 Bulletin 1995/46**

(60) Divisional application:
**01103999.7 / 1 103 544**

(73) Proprietor: **Ajinomoto Co., Inc.**
**Tokyo 104 (JP)**

(72) Inventors:
- **Makino, Shingo, c/o Central Research Labo. Kawasaki-ku, Kawasaki-shi, Kanagawa-ken (JP)**
- **Arisaka, Harumi, c/o Central Research Labo. Kawasaki-ku, Kawasaki-shi, Kanagawa-ken (JP)**
- **Yamamoto, Hiroshi, c/o Central Research Labo. Kawasaki-ku, Kawasaki-shi, Kanagawa-ken (JP)**
- **Shoji, Masataka, c/o Central Research Labo. Kawasaki-ku, Kawasaki-shi, Kanagawa-ken (JP)**
- **Yoshimoto, Ryota, c/o Central Research Labo. Kawasaki-ku, Kawasaki-shi, Kanagawa-ken (JP)**

(74) Representative: **Nicholls, Kathryn Margaret et al**
**MEWBURN ELLIS**
**York House**
**23 Kingsway**
**London WC2B 6HP (GB)**

(56) References cited:
**EP-A- 0 307 303** **EP-A- 0 371 805**
**EP-A- 0 406 739** **EP-A- 0 479 601**
**FR-A- 1 322 527**

- **CHEMICAL ABSTRACTS, vol. 120, no. 21, 23 May 1994, Columbus, Ohio, US; abstract no. 270113w, & JP-A-05 097 808 (AJINOMOTO KK.) 20 April 1993**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 682 015 B1

Printed by Jouve, 75001 PARIS (FR)

## Description

The present invention relates to novel uses of certain piperidine derivatives as $5HT_2$ antagonists and as anti-platelet agents, and to novel piperidine derivatives with corresponding activity.

It is considered that the thrombus participates greatly in ischemic disorders such as cardiac infarction and cerebral infarction. In particular, platelets play an important role in the formation of arterial thrombus. Known anti-platelet agents include arachidonic acid metabolism-inhibiting agents, platelet cyclic nucleoside-related agents and thromboxane receptor antagonists. Aspirin and ticlopidine have been clinically used. However, their effect is not sufficient and development of more effective agents is in demand.

On the other hand, it is known that serotonin (5HT), which is stored in α granules of platelets, is released by activation of the platelet caused by various stimuli. The released serotonin increases the calcium ion level in the cell via the serotonin 2 ($5HT_2$) receptor on the platelet membrane, resulting in aggregation of platelets. It is also considered that $5HT_2$ receptors existing in vascular smooth muscle participate in blood vessel contraction. Accordingly, the $5HT_2$ receptor antagonist is expected to have vasoconstriction inhibiting activity in addition to the platelet aggregation inhibiting activity and, therefore, there is a possibility that a potent anti-thrombus function is obtained with $5HT_2$ receptor antagonists.

The object of the present invention is to provide novel uses of certain piperidine compounds in $5HT_2$ antagonist and anti-platelet agent pharmaceutical preparations which potently and specifically inhibit the serotonin 2 receptor with low adverse side effects. Another aspect is novel piperidine compounds having such activity.

In the prior art, US-A-4891376 (equivalent to EP-A-307303) describes pyrimidinylaminoalkylpiperidine compounds of the formula

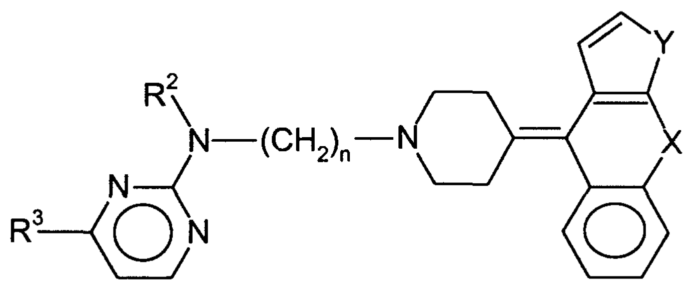

where

X is $(CH_2)_2$, -CH=CH- or $CH_2$-CO;
Y is -CH=CH or S
n is 2, 3 or 4
$R^1$ is H or halogen
$R^2$ is H or $C_{1-4}$ alkyl, and
$R^3$ is H or OH

and their pharmaceutical utility as antiserotoninergic agents at $5HT_2$ receptors; mentioned indications include treatment of CNS disorders, hypertension and platelet aggregation.

Our EP-A-479601 describes a range of piperidine derivatives for anti-arrhythmia use, including those of the general formula

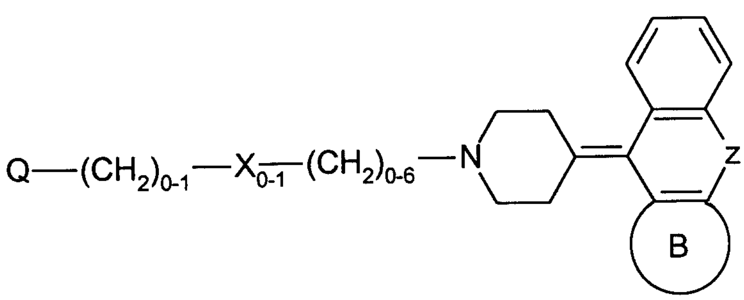

where Q is phenyl, cyclohexyl, piperidinyl, tetrahydropyranyl, pyridyl, pyrrolyl, thienyl, furyl, 1-hexyl or cyano;

X is any of a wide range of divalent entities including amide linkages;
Z may be (inter alia) -CH=CH-, $-S-CH_2$, S or $-(CH_2)_2-$;
B is benzene, pyridine or thiophene.

Compound 12 in EP-A-479601 is 1-(N-acetyl-4-piperidinyl)-3-[4-(5H-dibenzo[a,d]cyclohepten-5-ylidene)-1-piperidinyl] propane.

EP-A-406739 describes compounds of the formula

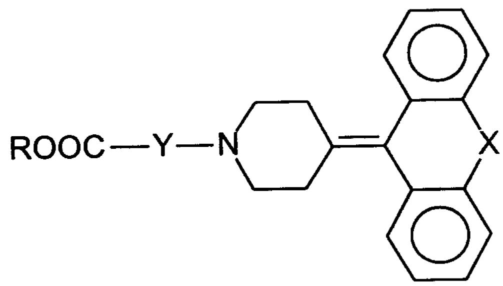

where

X is inter alia -CH=CH- or $-CH_2-CH_2-$;
Y is inter alia $C_{1-5}$ alkylene
R is H or lower alkyl

for pharmaceutical use as antihistaminic and anti-allergic agents.

Our EP-A-371805 describes the hypotensive use of piperidine derivatives, inter alia 1-cyclohexylmethyl-4-(5H-dibenzo[a,d]cyclohepten-5-ylidene)piperidine hydrochloride and similar (Examples 7-11).

In one aspect the present invention provides the use of a pharmaceutically-acceptable compound which is a piperidine derivative of general formula (I) or salt thereof as active ingredient in the manufacture of a medicament for use as a $5HT_2$ antagonist:

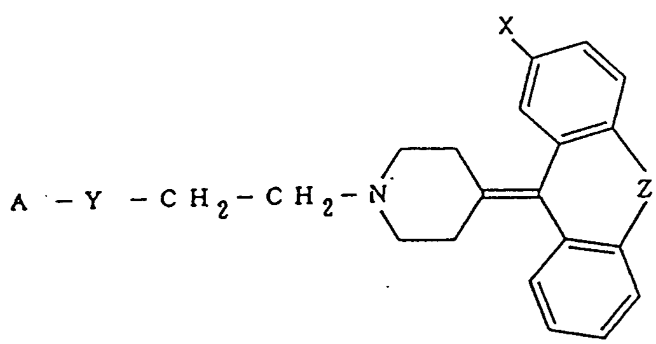

( I )

wherein A represents unsubstituted or substituted piperidyl, piperidino, morpholinyl, morpholino, thiomorpholinyl, thiomorpholino or piperazinyl group, or unsubstituted or substituted alkoxyl group having 1 to 8 carbon atoms,

X represents hydrogen atom or halogen atom,
Y represents one of the organic groups:

$$-CONH-, -NHCO-, -CONHCH_2-, -(CH_2)_n-, -COO-,$$

wherein n is an integer of from 0 to 4, provided that Y is not $-(CH_2)_n-$ when A is piperidyl, and
Z represents one of the organic groups: -CH=CH-, $-S-CH_2-$, -S-, $-CH_2-CH_2-$,

and wherein when A is a said substituted group, a substituent on A is one of the groups:

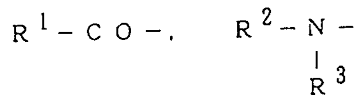

wherein $R^1$ is a hydrogen atom, an alkyl or alkoxy group having from 1 to 6 carbon atoms, an amino group which may be alkyl-substituted or an acylaminoalkyl group, and $R^2$ and $R^3$, which may be the same or different, each represent a hydrogen atom, an alkyl, acyl or alkoxycarbonyl group having from 1 to 6 carbon atoms, or an aminocarbonyl group which may be alkyl-substituted.

Illustrative examples of the substituents on A include formyl, acetyl, propionyl, butyryl, isobutyryl, valeryl, isovaleryl, pivaloyl, carbamoyl, N-methylcarbamoyl, N-ethylcarbamoyl, N-propylcarbamoyl, N,N-dimethylcarbamoyl, N,N-diethylcarbamoyl, N-formylglycyl, N-acetylglycyl, N-formyl-β-alanyl, N-acetyl-β-alanyl, N-methyl-N-formyl, N-methyl-N-acetyl, N-methyl-N-propionyl, N-ethyl-N-formyl, and N-ethyl-N-acetyl.

Preferable examples of Y in the general formula (I) include a group -CONH-, and preferable examples of Z include -CH=CH-.

Among the compounds represented by the general formula (I), the compounds wherein $R^1$ represents an alkyl or alkoxyl group having from 1 to 6 carbon atoms, an amino group which may be alkyl-substituted or an acylaminoalkyl group, $R^2$ and $R^3$, which may be the same or different, each represents a hydrogen atom, an alkyl, acyl or alkoxycarbonyl group having 1 to 6 carbon atoms, or an aminocarbonyl group which may be alkyl-substituted, and some others, are novel compounds that have not been references. See claims 9 and 14.

The piperidine derivative represented by the above general formula (I) may be prepared by a conventional method, for example, by the method described in JP-A-3/47168.

For example, 4-(5H-dibenzo[a,d]cyclohepten-5-ylidene)-1-(2-t-butoxycarbonylamino)ethyl)piperidine (3) included in the general formula (I) can be easily obtained by subjecting N-t-butoxycarbonyl-2-bromoethylamine (1) and 4-(5H-dibenzo[a,d]cyclohepten-5-ylidene)piperidine (2) to condensation reaction in the presence of a base such as triethylamine, as shown in the Reaction Scheme I.

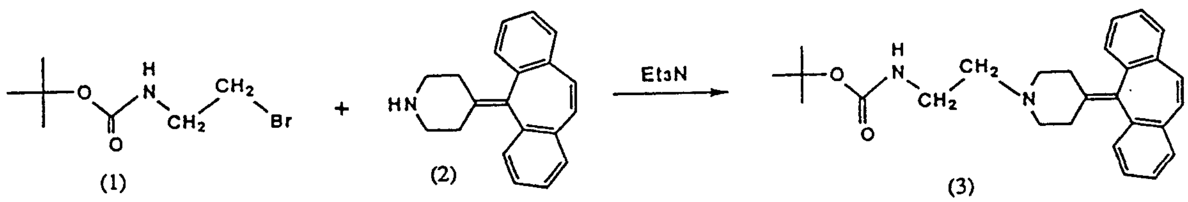

(1) (2) (3)

Similarly, 1-formyl-N-(2-(4-(5H-dibenzo[a,d]cyclohepten-5-ylidene)-1-piperidinyl))ethylisonipecotamide (6) included in the general formula (I) can be easily obtained by subjecting the compound (4), which is obtained by removing a t-butoxycarbonyl group from the compound (3) using 4 M hydrochloric acid/dioxane, etc., and 1-formylisonipecotic acid (5) to condensation reaction using a condensation agent such as 1-(3-dimethylaminopropyl)-3-ethylcarbodiimide, as shown in the Reaction Scheme II.

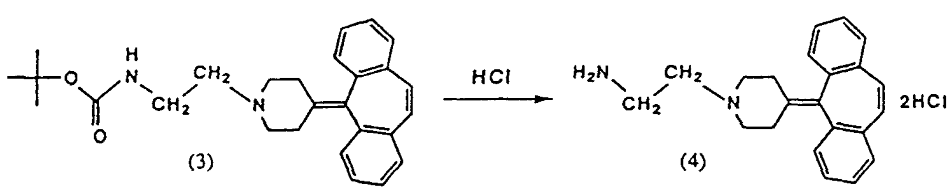

(3) (4)

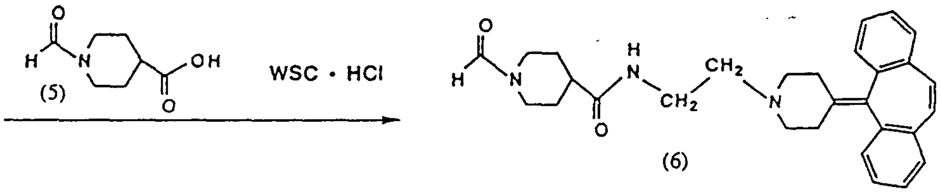


(6)

The reaction product obtained by these production methods is isolated and purified as a free compound or a salt thereof. Isolation and purification may be carried out by extraction, concentration, evaporation, crystallization, and various types of chromatography.

Examples of salts of such piperidine derivatives include acid addition salts with inorganic acid such as hydrochloric acid, hydrobromic acid, sulfuric acid, nitric acid, and phosphoric acid and with organic acids such as formic acid, acetic acid, lactic acid, salicylic acid, mandelic acid, citric acid, oxalic acid, maleic acid, fumaric acid, tartaric acid, tannic acid, malic acid, p-toluenesulfonic acid, methanesulfonic acid, and benzenesulfonic acid.

A piperidine derivative represented by the general formula (I) exhibits a serotonin 2 antagonistic activity and is useful as an agent for the treatment of ischemic disorders, thrombosis, obstruction, mental diseases (depression, anxiety), diabetic complication, arteriosclerosis, hypertension, migraine, microcirculation failure, and the like. In particular, as an anti-platelet agent, a piperidine derivative represented by the general formula (I) is useful as an agent for the treatment of various ischemic disorders, thrombosis, obstruction, angiitis, diabetic complication, arteriosclerosis, nephropathy, and ulcer, pain, rhigosis, etc. due to chronic arterial obstruction, and also can be used as a treating agent for improving various ischemia accompanying circulation failure, for preventing restenosis after surgical treatment of ischemic heart diseases, and for improving blood circulation.

When the piperidine derivative of the general formula (I) is used as a serotonin 2 antagonist or an anti-platelet agent, the administration route may be either oral or parenteral. The clinical dose may differ depending on the age, body weight, and condition of the patient and on the administration method, but the dose per adult per day is generally from 0.01mg to 500mg, preferably from 0.1mg to 50mg, in the case of oral administration and 1μg to 100mg, preferably from 0.01mg to 10mg, in the case of parenteral administration.

As the dosage form, usual dosage forms such as tablets, powders, sugar-coated preparations, capsules and solutions may be employed and such dosage forms can be prepared by the conventional method making use of usual pharmaceutical adjuvants.

Examples

The present invention will be further illustrated by way of Examples but the present invention should not be construed as being limited thereto.

Preparation procedure A

Synthesis of 1-methoxycarbonyl-N-(2-(4-(5H-dibenzo[a,d]cycl ohepten-5-ylidene)-1-piperidinyl))ethylisonipecotamide hydrochloride

Step 1

Synthesis of 2-t-Butoxycarbonylaminoethylbromide

2-Aminoethylbromide hydrobromide (35.77 g, 174.6 mmol) and di-t-butyl dicarbonate (22.80 g, 104.5 mmol) were added to a mixed solvent of 300 ml of diethyl ether and 300 ml of water. Then, sodium hydrogencarbonate (44.00 g, 523.7 mmol) was gradually added and the mixture was stirred at room temperature overnight. The diethyl ether layer was washed with 80 ml of 1N hydrochloric acid and then with 80 ml of a saturated aqueous sodium chloride solution, and dried over magnesium sulfate powder. The solvent was evaporated to obtain the titled compound.
Amount obtained: 21.57 g (96.25 mmol); Yield: 92%

Step 2

Synthesis of 4-(5H-dibenzo[a,d]cyclohepten-5-ylidene)-1-(2-t-butoxycarbonylamino)ethyl)piperidine

2-t-Butoxycarbonylaminoethylbromide (4.5 g, 20.1 mmol), 4-(5H-dibenzo[a,d]cyclohepten-5-ylidene)piperidine (2.7 g, 10.0 mmol), and triethylamine (4.2 ml, 30 mmol) were added to acetonitrile (300 ml), and the mixture was stirred on an oil bath at 50°C for 16 hours. The temperature was lowered to room temperature, the solvent was evaporated, and the residue was dissolved in 300 ml of ethyl acetate. After removing insoluble matters by filtration, the filtrate was washed with 100 ml of 1N hydrochloric acid, 100 ml of a 1N aqueous sodium hydroxide solution, and 100 ml of a saturated sodium chloride aqueous solution, and dried over magnesium sulfate powder. The solvent was evaporated and the residue was purified by silica gel column chromatography to obtain the titled compound. Amount obtained: 3.6 g (8.6 mmol); Yield: 86%

Step 3

Synthesis of 1-(2-aminoethyl)-4-(5H-dibenzo[a,d]cyclohepten-5-ylidene)piperidine dihydrochloride

4-(5H-Dibenzo[a,d]cyclohepten-5-ylidene)-1-(2-t-butoxycarbonylamino)ethyl)piperidine (8.47 g, 20.4 mmol) was dissolved in 100 ml of dichloromethane, and 100 ml of a 4N hydrochloric acid-dioxane solution was added thereto, followed by stirring at room temperature for 1 hour. The solvent was evaporated to obtain the titled compound (8.56 g).

Step 4

Synthesis of 1-t-butoxycarbonyl-N-(2-(4-(5H-dibenzo [a,d]cyclohepten-5-ylidene)-1-piperidinyl))ethylisonipecotamide

1-(2-Aminoethyl)-4-(5H-dibenzo[a,d]cyclohepten-5-ylidene)piperidine dihydrochloride (2.3 g, 6.0 mmol), 1-t-butoxycarbonylisonipecotic acid (1.6 g, 7.2 mmol), triethylamine (3.0 ml, 21.6 mmol) and 1-ethyl-3-(3-dimethylaminopropyl)carbodiimide hydrochloride (1.4 g, 7.2 mmol) were mixed, and the mixture was stirred at room temperature overnight. After evaporating the solvent, the residue was dissolved in 100 ml of dichloromethane, washed with 100 ml of 1N hydrochloric acid, 100 ml of a 1N aqueous sodium hydroxide solution, and 50 ml of a saturated aqueous sodium chloride solution. The solvent was evaporated and the residue was purified by silica gel chromatography to obtain the titled compound. Amount obtained: 2.0 g (3.8 mmol); Yield: 63%

Step 5

Synthesis of N-(2-(4-(5H-dibenzo[a,d]cyclohepten-5- ylidene)-1-piperidinyl))ethylisonipecotamide dihydrochloride

10 ml of 4N hydrochloric acid-dioxane solution was added to 1-t-butoxycarbonyl-N-(2-(4-(5H-dibenzo[a,d] cyclohepten-5-ylidene)-1-piperidinyl))ethylisonipecotamide (0.10 g, 0.185 mmol), and the mixture was stirred at room temperature for 1 hour. The solvent was evaporated to obtain the titled compound. Amount obtained: 0.093 g (0.186 mmol); Yield: 100%

Step 6

Synthesis of 1-methoxycarbonyl-N-(2-(4-(5H-dibenzo [a,d]cyclohepten-5-ylidene)-1-piperidinyl))ethylisonipecotamide hydrochloride

N-(2-(4-(5H-Dibenzo[a,d]cyclohepten-5-ylidene)-1-piperidinyl))ethylisonipecotamide dihydrochloride (0.59 g, 1.18 mmol) and triethylamine (0.8 ml, 5.70 mmol) were dissolved in 50 ml of dichloromethane, and methyl chloroformate (0.1 ml, 1.40 mmol) was added. The mixture was stirred for 1 hour and 100 ml of dichloromethane was added. The mixture was washed with 70 ml of water, 70 ml of a 1N aqueous sodium hydroxide solution, and 70 ml of a saturated aqueous sodium chloride solution, and purified by silica gel chromatography. The product obtained was converted into the hydrochloride form to give the titled compound. Amount obtained: 0.39 g (0.75 mmol); Yield: 63%

The compounds shown in Table 1 were produced in a similar manner as described in Preparation procedure A.

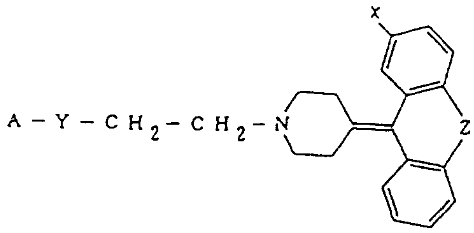

| Compound | A | Y | X | Z | pKi | $pIC_{50}$ |
|---|---|---|---|---|---|---|
| 1 | $CH_3OCO$- 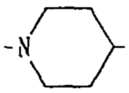 | -CONH- | H | -CH=CH- | 8.2 | 7.5 |
| 2 | H- 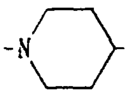 | | | | 8.6 | 7.3 |
| 3 | HCO- 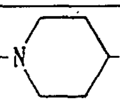 | | | | 8.4 | 7.2 |
| 4 | $CH_3CO$- 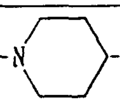 | | | | 8.5 | 7.2 |
| 5 | $(CH_3)_3COCO$- 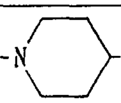 | | | | 8.0 | 6.5 |
| 6 | $H_2NCO$- 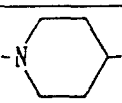 | | | | — | 6.9 |
| 7 | $(CH_3)_2NCO$- 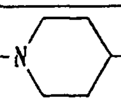 | | | | 8.5 | 6.7 |
| 8 | $CH_3CONHCH_2CO$- 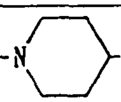 | | | | 7.9 | 6.6 |
| 9 | H- 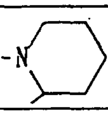 | | | | 7.8 | 7.1 |
| 10 | $CH_3CO$- 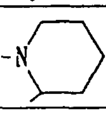 | | | | 8.8 | — |
| 11 | HCO- 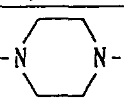 | | | | 8.8 | 7.0 |

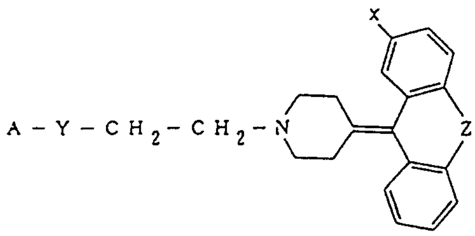

| | A | Y | X | Z | pKi | $pIC_{50}$ |
|---|---|---|---|---|---|---|
| 12 | $CH_3CH_2O-$ | -CONH-H | H | -CH=CH- | - | 7.6 |
| 13 | $(CH_3)_3CO-$ | | | | - | 7.0 |

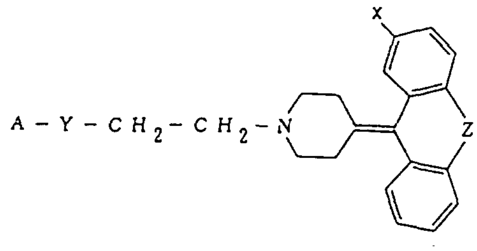

| | A | Y | X | Z | pKi | $pIC_{50}$ |
|---|---|---|---|---|---|---|
| 14 | 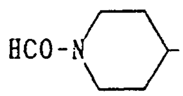 | $-CONHCH_2-$ | H | -CH=CH- | 8.3 | 6.4 |
| 15 | $(CH_3)_3CO-$ | | | | 8.8 | - |
| 16 | 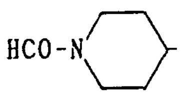 | -COO- | | | 8.3 | 7.4 |
| 17 | $(CH_3)_3CO-$ | -CONH- | | $-CH_2CH_2-$ | 8.3 | - |
| 18 | 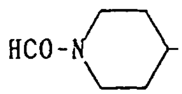 | | | | 8.9 | 5.8 |
| 19 | $(CH_3)_3CO-$ | -CONH- | | -S- | 9.1 | 7.3 |
| 20 | 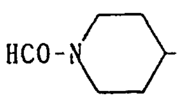 | | | | 9.2 | 7.1 |
| 21 | 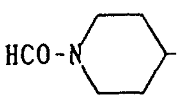 | | F | $-S-CH_2-$ | 7.1 | 5.6 |

Preparation procedure B

Synthesis of 1-(4-(4-(5H-dibenzo[a,d]cyclohepten-5-ylidene)-1-piperidinyl)butyl)morpholine

Step 1

Synthesis of 1-(4-oxo-4-morpholinobutyryl)-4-(5H-dibenzo[a,d]cyclohepten-5-ylidene)piperidine

In 50 ml of dichloromethane, 4-(5H-dibenzo[a,d]cyclohepten-5-ylidene)piperidine (0.27 g, 1.0 mmol), succinic anhydride (0.12 g, 1.2 mmol), and triethylamine (0.17 ml, 1.2 mmol) were stirred at room temperature overnight. Morpholine (0.14 ml, 1.6 mmol) and 1-ethyl-3- (3-dimethylaminopropyl)carbodiimide hydrochloride (0.27 g, 1.4 mmol) were added and the mixture was further stirred at room temperature for 8 hours. The reaction mixture was washed with 30 ml of 1N hydrochloric acid, 30 ml of a 1N aqueous sodium hydroxide solution, and 30 ml of a saturated aqueous sodium chloride solution, dried over magnesium sulfate powder, and purified by silica gel chromatography to obtain the titled compound. Amount obtained: 0.44 g (1.0 mmol); Yield: 100%

Step 2

Synthesis of 1-(4-(4-(5H-dibenzo[a,d]cyclohepten-5- ylidene)-1-piperidinyl)butyl)morpholine dihydrochloride

In tetrahydrofuran (60 ml), 1-(4-oxo-4-morpholinobutyryl)-4-(5H-dibenzo[a,d]cyclohepten-5-ylidene)piperidine (0.44 g, 1.00 mmol) was reacted with lithium aluminum hydride (0.38 g, 10.0 mol) at 0°C, and further treated in accordance with the conventional method to obtain the titled compound. Amount obtained: 0.32 g (0.66 mmol); Yield: 66%

The compounds shown in Table 2 were produced by the similar manner as described in Preparation procedure B.

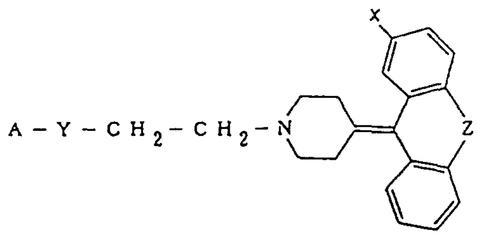

| | A | Y | X | Z | pKi | $pIC_{50}$ |
|---|---|---|---|---|---|---|
| 22 | 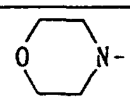 | $-(CH_2)_2-$ | H | -CH=CH- | 8.2 | 6.9 |
| 23 | 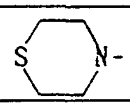 | | | | 8.7 | 6.6 |

Test Example 1

Binding affinity to the serotonin 2 receptor was evaluated using a bovine cerebral cortex membrane sample. 200 µl of 3 nM [$^3$H]-ketanserin and 200 µl of a test compound solution prepared by dissolving a test compound in 1.7% ethanol were added to 200 µl of a bovine membrane sample adjusted to 50 mg (wet weight) membrane/ml, followed by mixing. The mixture was incubated at 25°C for 30 minutes and filtered with a glass filter. The radioactivity trapped on the filter was measured with a liquid scintillation counter. Non-specific binding was defined by $10^{-6}$ M LY53857. The concentration of the test compound which inhibits 50% of the specific binding of [$^3$H]-ketanserin (i.e., $IC_{50}$ value) was obtained, and the Ki value was calculated in accordance with the following equation. The results are shown as the negative logarithm of the Ki value (i.e., pKi value).

$$Ki = \frac{IC_{50}}{1 + \frac{[L]}{Kd}}$$

In the equation, Ki indicates the dissociation constant and [L] indicates the concentration of [$^3$H]-ketanserin.

From the results in Tables 1 and 2, it is apparent that the piperidine derivatives of the present invention exhibit strong binding affinity to the serotonin 2 receptor.

Test Example 2

The anti-platelet effect due to the serotonin antagonistic activity was measured *in vitro* using platelets of SD rates (body weight about 300 to 400 g, male). Platelet-rich plasma (PRP) and platelet-poor plasma (PPP) were prepared from blood, obtained from aorta abdominalis of a rat under diethyl ether anesthesia, with 0.38% sodium citrate. The platelet concentration of PRP was adjusted to 5 x $10^8$ platelets/ml by adding PPP. Then, the test compound dissolved in 0.4% aqueous ethanol was added and the mixture incubated at 37°C for 3 minutes. The platelet aggregation induced

by addition of 0.5 μM or 0.8 μM adenosine diphosphate (ADP) + serotonin was measured as an increase in optical transmittance of the PRP. The concentration of the test compound which inhibits 50% of the increase in platelet aggregation which is obtained with serotonin without a test compound was measured, and its negative logarithm ($pIC_{50}$) was calculated. The results are shown in the Table 1 and 2. From these results, it is apparent that the piperidine compounds of the present invention potently inhibit platelet aggregation by serotonin.

Test Example 3

The anti-platelet effect due to serotonin antagonistic activity was measured *in vivo* using SD rats (body weight about 210 to 330 g, male). The test compound was dissolved or suspended in gum arabic and administered orally to the rats in a dose shown in Table 3. Two hours after the administration of the test compound, the rat was anaesthetized with diethyl ether. Platelet-rich plasma (PRP) and platelet-poor plasma (PPP) were prepared from blood, obtained from aorta abdominalis of the rat, with 0.38% sodium citrate. The platelet concentration of PRP was adjusted to $5 \times 10^8$ platelets/ml by adding PPP. Then, the PRP was incubated at 37°C for 3 minutes Platelet aggregation induced by addition of 0.7 μM adenosine diphosphate (ADP) + serotonin was measured as an increase in optical transmittance of the PRP. The aggregation incurred by addition of ADP alone and the maximum aggregation ratio by the simultaneous addition of ADP and serotonin were measured with respect to each group, and the increase in aggregation caused by serotonin was calculated. The increase in aggregation caused by serotonin in the gum arabic-administered group was taken as 100%, and the effect of the test compound was judged using as an index the increase in aggregation caused by the serotonin in the test compound-administered group (n = 3). The results are shown in Table 3.

Table 3

| Test compound | Amount of administration (mg/kg) | Increase in aggregation by serotonin (%) |
|---|---|---|
| gum arabic | - | 100 |
| compound of No. 3 | 0.1 | 75.7 |
| | 0.3 | 57.3 |
| | 1 | 24.3 |
| | 3 | 27 |
| | 10 | -2.7 |
| compound of No. 9 | 0.3 | 57.3 |
| compound of No. 17 | 0.3 | 50.7 |
| compound of No. 18 | 0.3 | 94.9 |
| compound of No. 38 | 0.3 | 82.4 |
| compound of No. 39 | 0.3 | 54.5 |
| compound of No. 41 | 0.3 | 91.5 |

From the results in the Table 3, it is apparent that the piperidine compounds of the present invention potently inhibit the platelet aggregation by serotonin even in the case of oral administration.

Test Example 4

The serotonin antagonistic activity in the central nervous system was evaluated by measuring the inhibiting effect on head twitch in mice induced by 5-hydroxytryptophan (5HTP). The test compound, in amounts of 1, 3, 10, or 30 mg, was dissolved in 100 ml of water and, 90 minutes before 5HTP administration, the solution (10 ml/kg body weight) was orally administered to an ICR mouse (body weight 27 to 32 g, male) fasted from the previous day. As a control, 5% gum arabic was used. Carbidopa (6 mg/kg) was subcutaneously administered and, after 15 minutes, 5HTP (180 mg/kg) was intraperitoneally administered. From 15 minutes after 5HTP administration, the number of head twitches occurring within 2 minutes was counted. The concentration of the test compound which inhibits 50% of the number of head twitches observed in the 5% arabic gum-administered group was obtained. The results are shown in Table 4.

Table 4

| Test compound | $ID_{50}$(mg/kg) |
|---|---|
| compound of No. 3 | 0.39 |
| cycloheptadine | 0.12 |

From the results in Table 4, it is apparent that the piperidine compound of the present invention has low effect on the central nerve system and is a highly safe compound.

## Claims

**1.** Use of a pharmaceutically-acceptable compound which is a piperidine derivative of general formula (I) or salt thereof as active ingredient in the manufacture of a medicament for use as a $5HT_2$ antagonist:

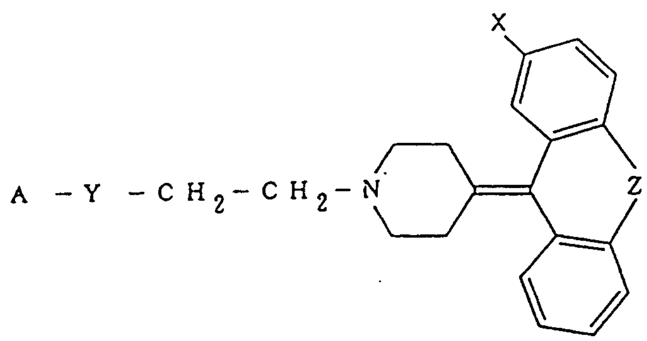

( I )

wherein A represents unsubstituted or substituted piperidyl, piperidino, morpholinyl, morpholino, thiomorpholinyl, thiomorpholino or piperazinyl group, or unsubstituted or substituted alkoxyl group having 1 to 8 carbon atoms,

X represents hydrogen atom or halogen atom,
Y represents one of the organic groups:

$$-CONH-, -NHCO-, -CONHCH_2-, -(CH_2)_n-, -COO-,$$

wherein n is an integer of from 0 to 4, provided that Y is not $-(CH_2)_n-$ when A is piperidyl, and
Z represents one of the organic groups:

$$-CH=CH-, -S-CH_2-, -S-, -CH_2-CH_2-,$$

and wherein when A is a said substituted group, a substituent on A is one of the groups:

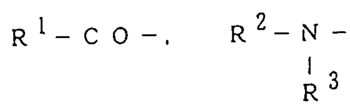

wherein $R^1$ is a hydrogen atom, an alkyl or alkoxy group having from 1 to 6 carbon atoms, an amino group which may be alkyl-substituted or an acylaminoalkyl group, and $R^2$ and $R^3$, which may be the same or different, each represent a hydrogen atom, an alkyl, acyl or alkoxycarbonyl group having from 1 to 6 carbon atoms, or

an aminocarbonyl group which may be alkyl-substituted.

**2.** Use according to claim 1 wherein A has a substituent which is formyl, acetyl, propionyl, butyryl, isobutyryl, valeryl, isovaleryl, pivaloyl, carbamoyl, N-methylcarbamoyl, N-ethylcarbamoyl, N-propylcarbamoyl, N,N-dimethylcarbamoyl, N,N-diethylcarbamoyl, N-formylglycyl, N-acetylglycyl, N-formyl-β-alanyl, N-acetyl-β-alanyl, N-methyl-N-formyl, N-methyl-N-acetyl, N-methyl-N-propionyl, N-ethyl-N-formyl, and N-ethyl-N-acetyl.

**3.** Use according to claim 1 or 2 wherein Y is -CONH-.

**4.** Use according to any one of the preceding claims, wherein Z is -CH=CH-.

**5.** Use according to claim 1 wherein the piperidine derivative is 1-formyl-N-(2-(4-(5H-dibenzo[a,d]cyclohepten-5-ylidene)-1-piperidinyl))ethylisonipecotamide.

**6.** Use according to any one of the preceding claims, wherein the medicament is for anti-platelet treatment.

**7.** Use according to any one of claims 1 to 5, in which the medicament is for the treatment of ischemic disorder, thrombosis, diabetic complication, arteriosclerosis, migraine or microcirculation failure.

**8.** Use according to claim 6 in which the medicament is for the treatment of ischemic disorder, thrombosis, obstruction, angiitis, diabetic complications, arteriosclerosis, nephropathy, ulcer, of pain or rhigosis due to chronic arterial obstruction, or is for preventing restenosis after surgical treatment of ischemic heart disease or for improving blood circulation.

**9.** A pharmaceutically-acceptable compound which is a piperidine derivative represented by the general formula (I), or salt thereof:

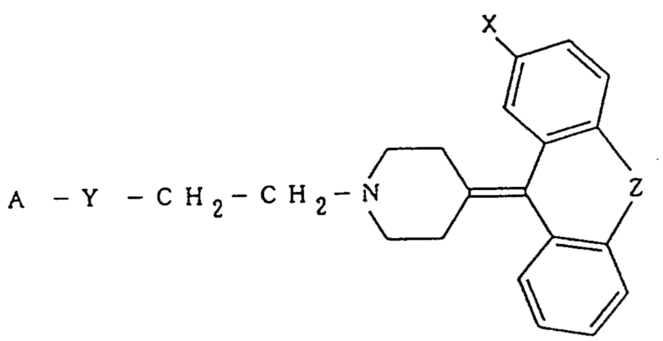

(I)

wherein A represents unsubstituted or substituted piperidyl, piperidino, morpholinyl, morpholino, thiomorpholinyl, thiomorpholino or piperazinyl group, or unsubstituted or substituted alkoxyl group having 1 to 8 carbon atoms, and when A is a said substituted group, a substituent on A is one of the groups:

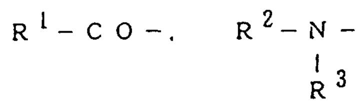

wherein $R^1$ represents an alkyl or alkoxy group having from 1 to 6 carbon atoms, an amino group which may be alkyl-substituted or an acylaminoalkyl group, $R^2$ and $R^3$, which may be the same or different, each represent a hydrogen atom, an alkyl, acyl or alkoxycarbonyl group having from 1 to 6 carbon atoms, or an aminocarbonyl group which may be alkyl-substituted; and X, Y and Z have the same meanings as in claim 1, including the proviso regarding Y and A.

**10.** A compound according to claim 9, wherein A has a substituent and said substituent is acetyl, propionyl, butyryl, isobutyryl, valeryl, isovaleryl, pivaloyl, carbamoyl, N-methylcarbamoyl, N-ethylcarbamoyl, N-propylcarbamoyl, N,N-dimethylcarbamoyl, N,N-diethylcarbamoyl, N-formylglycyl, N-acetylglycyl, N-formyl-β-alanyl, N-acetyl-β-alanyl,

N-methyl-N-formyl, N-methyl-N-acetyl, N-methyl-N-propionyl, N-ethyl-N-formyl or N-ethyl-N-acetyl.

**11.** A compound according to claim 9 or claim 10, wherein Y is -CONH-.

**12.** A compound according to any one of claims 9 to 11, wherein Z is -CH=CH-.

**13.** A compound according to claim 9 selected from 1-acetyl-N-(2-(4-(5H-dibenzo[a,d]cyclohepten-5-ylidene)-1-piperidinyl))ethylisonipecotamide, 1-t-butoxycarbonyl-N-(2-(4-(5H-dibenzo[a,d]cyclohepten-5-ylidene)-1-piperidinyl)) ethylisonipecotamide, 1-(N,N-dimethylcarbamoyl)-N-(2-(4-(5H-dibenzo[a,d]cyclohepten-5-ylidene)-1-piperidinyl)) ethylisonipecotamide, 1-(N-acetylglycyl)-N-(2-(4-(5H-dibenzo[a,d]cyclohepten-5-ylidene)-1-piperidinyl))ethylisonipecotamide, N-(2-(4-(5H-dibenzo [a,d] cyclohepten-5-ylidene) -1-piperidinyl)) ethyl- (N-acetyl)pipecolamide, 4-(5H-dibenzo[a,d]cyclohepten-5-ylidene)-1-(2-ethoxycarbonylamino)ethyl)piperidine, 4-(5H-dibenzo[a,d]cyclohepten-5-ylidene)-1-(2-t-butoxycarbonylamino)ethyl)piperidine, 4-(5H-dibenzo[a,d]cyclohepten-5-ylidene)-1-(3-t-butoxycarbonylaminopropyl)piperidine, 4-(10,11-dihydro-5H-dibenzo[a,d]cyclohepten-5-ylidene)-1-(2-t-butoxycarbonylamino)ethyl)piperidine, 4-(9-thioxanthylidene)-1-((2-t-butoxycarbonylamino)ethyl)piperidine, 1-(4-(4-(5H-dibenzo[a,d]cyclohepten-t-ylidene)-1-piperidinyl)butyl)morpholine, 1-(4-(4-(5H-dibenzo[a,d]cyclohepten-5-ylidene)-1-piperidinyl)butyl)thiomorpholine.

**14.** A compound selected from

1-formyl-4-((2-(4-(5H-dibenzo[a,d]cyclohepten-5-ylidene)-1-piperidinyl))ethylcarbamoyl)piperazine,
1-formyl-N-(3-(4-(5H-dibenzo[a,d]cyclohepten-5-ylidene)-1-piperidinyl))propylisonipecotamide,
1-formyl-isonipecotic acid 2-(4-(5H-dibenzo[a,d]cyclohepten-5-ylidene)-1-piperidinyl))ethyl ester,
1-formyl-N-(2-(4-(10,11-dihydro-5H-dibenzo[a,d]cyclohepten-5-ylidene)-1-piperidinyl))ethylisonipecotamide,
1-formyl-N-(2-(4-(9-thioxanthylidene)-1-piperidinyl))ethylisonipecotamide, and
1-formyl-N-(2-(4-(11H-dibenzo[b,e]thiepin-2-fluoro-11-ylidene)-1-piperidinyl))ethylisonipecotamide.

**Patentansprüche**

**1.** Verwendung einer pharmazeutisch geeigneten Verbindung, die ein Piperidinderivat der allgemeinen Formel (I) oder ein Salz davon ist, als aktiver Bestandteil bei der Herstellung eines Arzneimittels für die Verwendung als $5HT_2$-Antagonist:

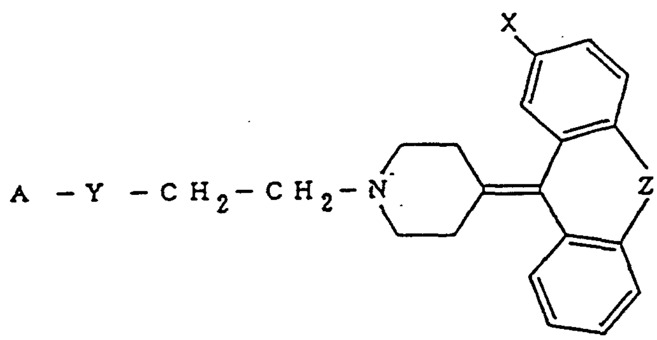

( I )

worin A eine unsubstituierte oder substituierte Piperidyl-, Piperidino-, Morpholinyl-, Morpholino-, Thiomorpholinyl-, Thiomorpholino- oder Piperazinylgruppe oder eine unsubstituierte oder substituierte Alkoxygruppe mit 1 bis 8 Kohlenstoffatomen darstellt,

X ein Wasserstoffatom oder ein Halogenatom ist,
Y eine der organischen Gruppen

-CONH-, -NHCO-, $-CONHCH_2-$, $-(CH_2)_n-$, -COO- darstellt,

worin n eine ganze Zahl von 0 bis 4 ist, mit der Maßgabe, daß Y nicht $-(CH_2)_n-$ ist, wenn A Piperidyl ist, und

Z eine der organischen Gruppen

$$-CH=CH-, -S-CH_2-, -S-, -CH_2-CH_2- \text{ darstellt,}$$

und worin, wenn A eine substituierte Gruppe ist, ein Substituent an A eine der Gruppen

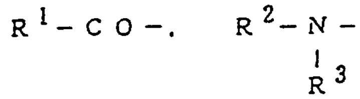

ist,
worin $R^1$ ein Wasserstoff, eine Alkylgruppe oder Alkoxygruppe mit 1 bis 6 Kohlenstoffatomen, eine Aminogruppe, die mit einer Alkylgruppe substituiert sein kann, oder eine Acylaminoalkylgruppe ist, und $R^2$ und $R^3$, die gleich oder verschieden sein können, jeweils ein Wasserstoffatom, eine Alkyl-, Acyl- oder Alkoxycarbonylgruppe mit 1 bis 6 Kohlenstoffatomen oder eine Aminocarbonylgruppe darstellen, die mit einer Alkylgruppe substituiert sein kann.

**2.** Verwendung nach Anspruch 1, worin A einen Substituenten hat, der Formyl, Acetyl, Propionyl, Butyryl, Isobutyryl, Valeryl, Isovaleryl, Pivaloyl, Carbamoyl, N-Methylcarbamoyl, N-Ethylcarbamoyl, N-Propylcarbamoyl, N,N-Dimethylcarbamoyl, N,N-Diethylcarbamoyl, N-Formylglycyl, N-Acetylglycyl, N-Formyl-β-alanyl, N-Acetyl-β-alanyl, N-Methyl-N-formyl, N-Methyl-N-acetyl, N-Methyl-N-propionyl, N-Ethyl-N-formyl oder N-Ethyl-N-acetyl ist.

**3.** Verwendung nach Anspruch 1 oder 2, worin Y -CONH-ist.

**4.** Verwendung nach einem der vorstehenden Ansprüche, worin Z -CH=CH- ist.

**5.** Verwendung nach Anspruch 1, wobei das Piperidinderivat 1-Formyl-N-(2-(4-(5H-dibenzo[a,d]cyclohepten-5-yliden)-1-piperidinyl))ethylisonipecotamid ist.

**6.** Verwendung nach einem der vorstehenden Ansprüche, wobei das Medikament ein Medikament für die Anti-Plättchenbehandlung ist.

**7.** Verwendung nach einem der Ansprüche 1 bis 5, wobei das Medikament ein Medikament für die Behandlung von ischämischen Erkrankungen, Thrombose, Diabeteskomplikationen, Arteriosklerose, Migräne oder Versagen des Mikrokreislaufes ist.

**8.** Verwendung nach Anspruch 6, wobei das Medikament ein Medikament für die Behandlung von ischämischen Erkrankungen, Thrombose, Verschlüssen, Gefäßentzündung, Diabeteskomplikationen, Arteriosklerose, Nierenleiden, Geschwüren, Schmerzen oder Kälteempfinden aufgrund von chronischem Arterienverschluß oder ein Arzneimittel für die Verhinderung von Restenosis nach chirurgischer Behandlung einer ischämischen Herzkrankheit oder für die Verbesserung der Blutzirkulation ist.

**9.** Pharmazeutisch geeignete Verbindung, die ein Piperidinderivat der allgemeinen Formel (I) ist, oder ein Salz davon:

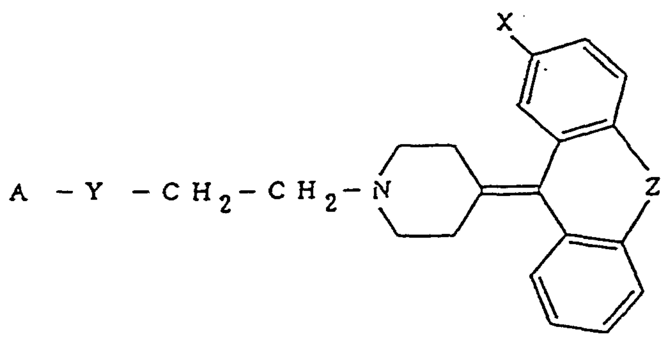

(I)

worin A eine unsubsisutierte oder substituierte Piperidyl-, Piperidino-, Morpholinyl-, Morpholino-, Thiomorpholinyl-, Thiomorpholino- oder Piperazinylgruppe oder eine unsubstituierte oder substituierte Alkoxygruppe mit 1 bis 8 Kohlenstoffatomen darstellt, und wenn A eine substituierte Gruppe ist, ein Substituent an A eine der Gruppen

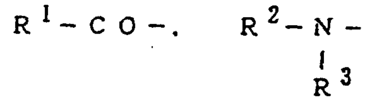

ist,
worin $R^1$ eine Alkyl- oder Alkoxygrupe mit 1 bis 6 Kohlenstoffatomen, eine Aminogruppe, die mit einer Alkylgruppe substituiert sein kann, oder eine Acylaminoalkylgruppe darstellt, $R^2$ und $R^3$, die gleich oder unterschiedlich sein können, jeweils ein Wasserstoffatom, eine Alkyl-, Acyl- oder Alkoxycarbonylgruppe mit 1 bis 6 Kohlenstoffatomen oder eine Aminocarbonylgruppe, die mit einer Alkylgruppe substituiert sein kann, darstellen, und X, Y und Z dieselbe Bedeutung wie in Anspruch 1 haben, einschließlich der Maßgabe betreffend Y und A.

**10.** Verbindung nach Anspruch 9, worin A einen Substituenten hat, der Acetyl, Propionyl, Butyryl, Isobutyryl, Valeryl, Isovaleryl, Pivaloyl, Carbamoyl, N-Methylcarbamoyl, N-Ethylcarbamoyl, N-Propylcarbamoyl, N,N-Dimethylcarbamoyl, N,N-Diethylcarbamoyl, N-Formylglycyl, N-Acetylglycyl, N-Formyl-β-alanyl, N-Acetyl-β-alanyl, N-Methyl-N-formyl, N-Methyl-N-acetyl, N-Methyl-N-propionyl, N-Ethyl-N-formyl oder N-Ethyl-N-acetyl ist.

**11.** Verbindung nach Anspruch 9 oder 10, worin Y -CONH- ist.

**12.** Verbindung nach einem der Ansprüche 9 bis 11, worin Z -CH=CH- ist.

**13.** Verbindung nach Anspruch 9, die unter 1-Acetyl-N-(2-(4-(5H-dibenzo[a,d]cyclohepten-5-yliden)-1-piperidinyl))-ethylisonipecotamid, 1-t-Butoxycarbonyl-N-(2-(4-(5H-dibenzo-[a,d]cyclohepten-5-yliden)-1-piperidinyl))ethylisonipecotamid, 1-(N,N-Dimethylcarbamoyl)-N-(2-(4-(5H-dibenzo[a,d]-cyclohepten-5-yliden)-1-piperidinyl))ethylisonipecotamid, 1-(N-Acetylglycyl)-N-(2-(4-(5H-dibenzo[a,d]cyclohepten-5-yliden)-1-piperidinyl))ethylisonipecotamid, N-(2-(4-(5H-dibenzo[a,d]cyclohepten-5-yliden)-1-piperidinyl))ethyl-(N-acetyl)pipecolamid, 4-(5H-Dibenzo [a,d]cyclohepten-5-yliden)-1-(2-ethoxycarbonylamino)ethyl)piperidin, 4-(5H-Dibenzo-[a,d]cyclohepten-5-yliden)-1-(2-t-Butoxycarbonylamino)-ethyl)piperdin, 4-(5H-Dibenzo[a,d]cyclohepten-5-yliden)-1-(3-t-butoxycarbonylaminopropyl)piperidin, 4-(10,11-Dihydro-5H-dibenzo[a,d]cyclohepten-5-yliden)-1-(2-t-butoxycarbonyl-amino)ethyl) piperidin, 4-(9-Thioxanthyliden)-1-((2-t-butoxy-carbonylamino)ethyl)piperidin, 1-(4-(4-(5H-dibenzo[a,d]cyclo-hepten-5-yliden)-1-piperidinyl)butyl)morpholin und 1-(4-(4-(5H-Dibenzo[a,d]cyclohepten-5-yliden)-1-piperidinyl)butyl)-thiomorpholin ausgewählt ist.

**14.** Verbindung, die unter

1-Formyl-4-((2-(4-(5H-dibenzo[a,d]cyclohepten-5-yliden)-1-piperidinyl))ethylcarbamoyl)piperazin,
1-Formyl-N-(3-(4-(5H-dibenzo[a,d]cyclohepten-5-yliden)-1-piperidinyl))propylisonipecotamid,
1-Formyl-isonipecotinsäure-2-(4-(5H-dibenzo[a,d]cyclohepten-5-yliden)-1-piperidinyl))ethylester,
1-Formyl-N-(2-(4-(10,11-dihydro-5H-dibenzo[a,d]cyclohepten-5-yliden)-1-piperidinyl))ethylisonipecotamid,

1-Formyl-N-(2-(4-(9-thioxanthyliden)-1-piperidinyl))ethylisonipecotamid, und 1-Formyl-N-(2-(4-(11H-dibenzo[b,e]thiepin-2-fluor-11-yliden)-1-piperidinyl))ethylisonipecotamid ausgewählt ist.

## Revendications

**1.** Utilisation d'un composé pharmaceutiquement acceptable qui est un dérivé de pipéridine de formule générale (I) ou un de ses sels en tant que principe actif dans la fabrication d'un médicament utile en tant qu'antagoniste de $5HT_2$ :

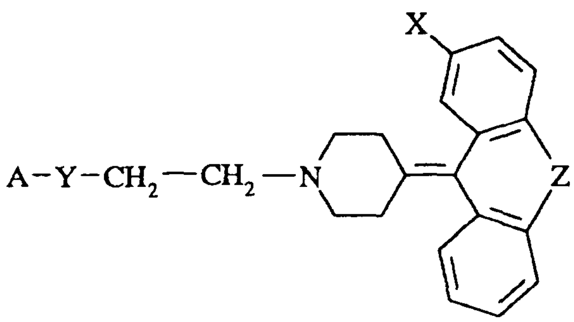

(I)

où A représente un groupe pipéridyle, pipéridino, morpholinyle, morpholino, thiomorpholinyle, thiomorpholino ou pipérazinyle, non substitué ou substitué, ou un groupe alcoxy ayant 1 à 8 atomes de carbone non substitué ou substitué,

X représente un atome d'hydrogène ou un atome d'halogène,
Y représente un des groupes organiques :

$$-CONH-, -NHCO-, -CONHCH_2-, -(CH_2)_n-, -COO-,$$

où n est un entier de 0 à 4, sous réserve que Y ne soit pas $-(CH_2)_n-$ si A est pipéridyle et
Z représente un des groupes organiques :

$$-CH=CH-, -S-CH_2-, -S-, -CH_2-CH_2-,$$

et où, si A est un groupe dit substitué, un substituant sur A est un des groupes :

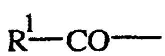 , 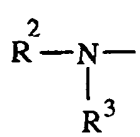

où $R^1$ représente un atome d'hydrogène, un groupe alkyle ou alcoxy ayant 1 à 6 atomes de carbone, un groupe amino pouvant être substitué par alkyle ou un groupe acylaminoalkyle et $R^2$ et $R^3$, qui peuvent être identiques ou différents, représentent chacun un atome d'hydrogène, un groupe alkyle, acyle ou alcoxycarbonyle ayant 1 à 6 atomes de carbone ou un groupe aminocarbonyle pouvant être substitué par alkyle.

**2.** Utilisation selon la revendication 1, dans laquelle A comporte un substituant qui est formyle, acétyle, propionyle, butyryle, isobutyryle, valéryle, isovaléryle, pivaloyle, carbamoyle, N-méthylcarbamoyle, N-éthylcarbamoyle, N-propylcarbamoyle, N,N-diméthylcarbamoyle, N,N-diéthylcarbamoyle, N-formylglycyle, N-acétylglycyle, N-formyl-β-alanyle, N-acétyl-β-alanyle, N-méthyl-N-formyle, N-méthyl-N-acétyle, N-méthyl-N-propionyle, N-éthyl-N-formyle et N-éthyl-N-acétyle.

3. Utilisation selon la revendication 1 ou 2, dans laquelle Y est -CONH-.

4. Utilisation selon l'une quelconque des revendications précédentes, dans laquelle Z est -CH=CH-.

5. Utilisation selon la revendication 1, dans laquelle le dérivé de la pipéridine est la 1-fonnyl-N-(2-(4-(5H-dibenzo[a, d]cycloheptén-5-ylidène)-1-pipéridinyl))éthylisonipécotamide.

6. Utilisation selon l'une quelconque des revendications précédentes, dans laquelle le médicament est destiné à un traitement anti-plaquettaire.

7. Utilisation selon l'une quelconque des revendications 1 à 5, dans laquelle le médicament est destiné au traitement d'un trouble ischémique, d'une thrombose, d'une complication diabétique, d'une artériosclérose, d'une migraine ou d'un trouble de la microcirculation.

8. Utilisation selon la revendication 6, dans laquelle le médicament est destiné au traitement d'un trouble ischémique, d'une thrombose, d'une obstruction, d'une angiite, de complications diabétiques, d'une artériosclérose, d'une néphropathie, d'un ulcère, d'une douleur ou d'une sensibilité au froid due à une obstruction artérielle chronique ou est destiné à la prévention d'une resténose après traitement chirurgical d'une cardiopathie ischémique ou à l'amélioration de la circulation sanguine.

9. Composé pharmaceutiquement acceptable qui est un dérivé de pipéridine représenté par la formule générale (I) ou un de ses sels :

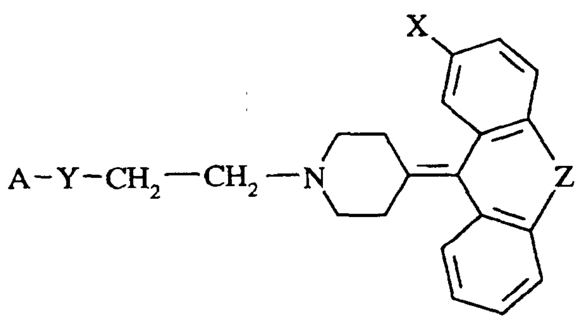

(I)

où A représente un groupe pipéridyle, pipéridino, morpholinyle, morpholino, thiomorpholinyle, thiomorpholino ou pipérazinyle, non substitué ou substitué, ou un groupe alcoxy ayant 1 à 8 atomes de carbone non substitué ou substitué, et si A est un groupe dit substitué, un substituant sur A est un des groupes :

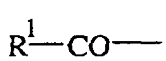

,

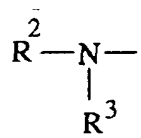

où $R^1$ représente un groupe alkyle ou alcoxy ayant 1 à 6 atomes de carbone, un groupe amino pouvant être substitué par alkyle ou un groupe acylaminoalkyle et $R^2$ et $R^3$, qui peuvent être identiques ou différents, représentent chacun un atome d'hydrogène, un groupe alkyle, acyle ou alcoxycarbonyle ayant 1 à 6 atomes de carbone ou un groupe aminocarbonyle pouvant être substitué par alkyle ; et
X, Y et Z ont les mêmes définitions que dans la revendication 1, la réserve concernant Y et A y compris.

10. Composé selon la revendication 9, dans lequel A comporte un substituant et ledit substituant est acétyle, propionyle, butyryle, isobutyryle, valéryle, isovaléryle, pivaloyle, carbamoyle, N-méthylcarbamoyle, N-éthylcarbamoyle, N-propylcarbamoyle, N,N-diméthylcarbamoyle, N,N-diéthylcarbamoyle, N-formylglycyle, N-acétylglycyle, N-formyl-β-alanyle, N-acétyl-β-alanyle, N-méthyl-N-formyle, N-méthyl-N-acétyle, N-méthyl-N-propionyle, N-éthyl-N-formyle ou N-éthyl-N-acétyle.

11. Composé selon la revendication 9 ou la revendication 10, dans lequel Y est -CONH-.

**12.** Composé selon l'une quelconque des revendications 9 à 11, dans lequel Z est -CH=CH-.

**13.** Composé selon la revendication 9, choisi parmi la 1-acétyl-N-(2-(4-(5H-dibenzo[a,d]cycloheptén-5-ylidène)-1-pipéridinyl))éthylisonipécotamide, la 1-t-butoxycarbonyl-N-(2-(4-(5H-dibenzo[a,d]cycloheptén-5-ylidène)-1-pipéridinyl))éthylisonipécotamide, la 1-(N,N-diméthylcarbamoyl)-N-(2-(4-(5H-dibenzo[a,d]cycloheptén-5-ylidène)-1-pipéridinyl))-éthylisonipécotamide, la 1-(N-acétylglycyl)-N-(2-(4-(5H-dibenzo[a,d]-cycloheptén-5-ylidène)-1-pipéridinyl))éthylisonipécotamide, la N-(2-(4-(5H-dibenzo[a,d]cycloheptén-5-ylidène)-1-pipéridinyl))éthyl(N-acétyl)-pipécolamide, la 4-(5H-dibenzo[a,d]cycloheptén-5-ylidène)-1-(2-éthoxycarbonylamino)éthyl)pipéridine, la 4-(5H-dibenzo[a,d]cycloheptén-5-ylidéne)-1-(2-t-butoxycarbonylamino)éthyl)pipéridine, la 4-(5H-dibenzo[a,d]-cycloheptén-5-ylidène)-1-(3-t-butoxycarbonylaminopropyl)pipéridine, la dihydro-5H-dibenzo[a,d]cycloheptén-5-ylidène)-1-(2-t-butoxycarbonylamino)-éthyl)pipéridine, la 4-(9-thioxanthylidène)-1-(2-(t-butoxycarbonylamino)-éthyl)pipéridinc, la 1-(4-(4-(5H-dibenzo[a,d]cycloheptén-5-ylidène)-1-pipéridinyl)butyl)morpholine, la 1-(4-(4-(5H-dibenzo[a,d]cycloheptén-5-ylidène)-1-pipéridinyl)butyl)thiomorpholine.

**14.** Composé choisi parmi la 1-formyl-4-((2-(4-(5H-dibenzo[a,d]cycloheptén-5-ylidène)-1-pipéridinyl))éthylcarbamoyl) pipérazine, la 1-formyl-N-(3-(4-(5H-dibenzo[a,d]cycloheptén-5-ylidène)-1-pipéridinyl))-propylisonipécotamide, l'ester de 2-(4-(5H-dibenzo[a,d]cycloheptén-5-ylidène)-1-pipéridinyl))éthyle de l'acide 1-formylisonipécotique, la 1-formyl-N-(2-(4-(10,11-dihydro-5H-dibenzo[a,d]-cycloheptén-5-ylidène)-1-pipéridinyl))éthylisonipécotamide, la 1-formyl-N-(2-(4-(9-thioxanthylidène)-1-pipéridinyl))éthylisonipécotamide et la 1-formyl-N-(2-(4-(11H-dibenzo[b,e] thiépin-2-fluoro-11-ylidène)-1-pipéridinyl))éthylisonipécotamide.